# FASCICULE DE BREVET EUROPEEN

(11) **EP 0 517 589 B1**
(45) Date de publication et mention de la délivrance du brevet: **27.12.1996**
(21) Numéro de dépôt: 92401521.7
(22) Date de dépôt: 04.06.1992
(51) Int. Cl.: C07K 5/02, A61K 38/06, A61K 38/07

(54) **Dérivés de tachykinines, leur procédé de préparation et les compositions pharmaceutiques qui les contiennent**
Tachykininderivate, deren Herstellung und diese enthaltende pharmazeutische Zusammensetzungen
Tachykinin derivatives, their preparation and pharmaceutical compositions containing them

(30) Priorité: 04.06.1991 FR 9106721
(43) Date de publication de la demande: 09.12.1992
(60) Demande divisionnaire: 95200770.6
(73) Titulaire: ADIR ET COMPAGNIE, F-92415 Courbevoie Cédex (FR)
(72) Inventeur: Fauchere, Jean-Luc, F-92210 Saint-Cloud (FR); Kucharczyk, Nathalie, F-75013 Paris (FR); Morris, Angela D., F-78220 Viroflay (FR); Paladino, Joseph, F-78700 Conflans Sainte-Honorine (FR); Bonnet, Jacqueline, F-75013 Paris (FR); Thurieau, Christophe, F-92100 Boulogne sur Seine (FR)

(56) Documents cités:
- EP-A- 0 333 174
- EP-A- 0 333 174
- EP-A- 0 394 989
- EP-A- 0 394 989
- DD-A- 150 199
- CHEMICAL ABSTRACTS, vol. 94, 1981, Columbus, Ohio, US; abstract no. 175484d, I.SCHOEN C.S. 'FORMATION OF IMIDES IN STRONGLY ACIDIC MEDIA AND PARTICIPATION OF THE IMIDE IN TRANSFORMATION TO PIPERAZINE-2,5-DIONE DERIVATIVES IN NEUTRAL MEDIA' page 770 ;

## Description

La présente invention concerne de nouveaux peptides et pseudopeptides dérivés de tachykinines, leur procédé de préparation et les compositions pharmaceutiques qui les contiennent.

Les tachykinines forment une famille de peptides produisant des contractions rapides des fibres musculaires lisses, par opposition aux contractions lentes développées par les bradykinines. La substance P, la neurokinine A et la neurokinine B constituent les principales tachykinines endogènes correspondant respectivement aux récepteurs NK₁, NK₂ et NK₃.

De nombreux peptides antagonistes de tachykinines ont été décrits dans la littérature. C'est le cas par exemple des composés décrits dans les brevets EP-A-333174, EP-A-394989. Le brevet DD-A-150199 décrit l'introduction d'une partie cyclique dipeptidique sur le groupement N-terminal d'un peptide dérivé de la substance P.

La présente invention a pour objet des peptides synthétiques, qui, outre le fait qu'ils soient nouveaux, se sont montrés particulièrement intéressants par l'intensité de leurs propriétés pharmacologiques. Ils possèdent non seulement des propriétés antagonistes puissantes vis-à-vis des récepteurs aux tachykinines et plus particulièrement sélectives et intenses vis-à-vis des récepteurs NK₁ c'est-à-dire de substance P. Ces propriétés les rendent utilisables plus particulièrement dans le traitement de la douleur, de l'inflammation, des troubles gastro-intestinaux, de l'asthme, des allergies et des maladies du système nerveux central.

L'invention concerne plus particulièrement de nouveaux dérivés peptidiques répondant à la formule générale (I) : 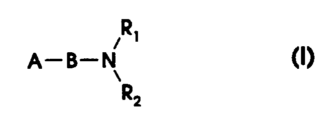 dans laquelle :
- R₁ et R₂: identiques ou différents représentent un atome d'hydrogène, un groupement alkyle (C₁-C₆) linéaire ou ramifié, un groupement cycloalkyle (C₃-C₇) ou un groupement benzyle,
- B: représente un résidu d'amino-acide aromatique,
- A: représente un résidu peptidique de formule : 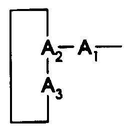
dans lequel :
- A₁: représente une liaison, un résidu 2-azabicyclo[2.2.2]octane-3-carbonyl (Abo), leucine (Leu), β-naphtylalanine (Nal), tryptophane (Trp), tryptophane protégé par un radical Q (Trp(Q)),
Q représentant le radical

-X-(CH₂)ₙ-R'

dans lequel
X représente une liaison, -CO-, ou -COO-,
n est un entier de 0 à 10,
R' est un atome d'hydrogène, un radical alkyle (C₁-C₁₀) linéaire ou ramifié, benzyle, 9-fluorénylméthyle, -NH₂, -COOH ou -COOR" (R" = alkyl (C₁-C₆) linéaire ou ramifié),
- A₂: représente un résidu acide aspartique (Asp),
- A₃: représente un résidu (1,2,3,4)-tétrahydroisoquinoléine-3-carbonyl (Tic), 2-aza-bicyclo[2.2.2]octane-3-carbonyl (Abo), ou méthylphénylalanine (MePhe),

étant entendu que la liaison peptidique (-CO-NH-) entre A₁ et A₂ ou entre A₂ et B dans le cas où A₁ est une liaison, peut être remplacée par une liaison pseudopeptidique choisie parmi -CH₂-NH- ou -CH₂-S-,
leurs énantiomères, diastéréoisomères et épimères ainsi que leurs sels d'addition à un acide ou à une base pharmaceutiquement acceptable,
étant entendu que chaque amino-acide de la séquence peptidique est optiquement pur et que le carbone α de chaque amino-acide peut être de configuration D ou L.

Par résidu d'amino-acide aromatique, on entend plus particulièrement résidu phénylalanine (Phe), tyrosine (Tyr), (1,2,3,4)-tétrahydroisoquinoléine-3-carbonyl (Tic), tryptophane (Trp), tryptophane protégé par un radical formyle (Trp (CHO)) ou pyridinylalanine (Pya).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend aussi au procédé de préparation des composés de formule (I) qui peuvent être obtenus par différentes méthodes telles que la synthèse séquentielle sur phase solide, la synthèse enzymatique, la synthèse génétique par clonage et expression des gènes dans des bactéries transformées ou par des combinaisons diverses de ces techniques.

Les peptides de la présente invention sont généralement obtenus par couplage en solution de fragments peptidiques sélectivement protégés, qui peuvent être préparés soit sur phase solide, soit en solution.

Les méthodes générales de la synthèse des peptides sur phase solide ont été décrites par B.W. ERICKSON et R.B. MERRIFIELD ("The Proteins", Solid-Phase Peptide Synthesis, 3ème édition, 257-527, 1976).

Plus spécifiquement, le procédé de préparation des composés de l'invention suit la méthode de synthèse et de couplage des fragments en solution, bien adaptée à la taille et aux modifications des composés.

Ce procédé est caractérisé en ce que l'on fait réagir un amide de formule (II), optiquement pur : 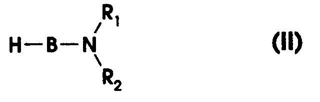 dans laquelle B, R₁ et R₂ ont la même signification que dans la formule (I), avec un deuxième amino-acide, optiquement pur, dont la fonction amine N-terminale est protégée, de formule (III):

P₁-A₁-OH (III)

dans laquelle :
P₁ représente un groupement protecteur choisi parmi benzyl-oxycarbonyl (Z), tert-butyloxycarbonyl (Boc), 9-fluorényl-méthylcarbonyl (Fmoc),
A₁ a la même signification que dans la formule (I),
en présence d'un réactif de couplage classique de la synthèse peptidique choisi parmi le couple dicyclohexylcarbodiimide (DCC) - hydroxybenzo-triazole (HOBT), le benzotriazol-1-oxytris (diméthylamino) phosphonium hexafluorophosphate (BOP) ou encore le diphénylphosphorylazide (DPPA),
pour conduire, après déprotection sélective de la fonction amine N-terminale, au composé de formule (IV) : 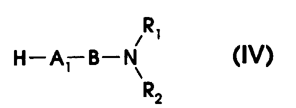 dans laquelle A₁, B, R₁ et R₂ ont les mêmes significations que dans la formule (I),
composé de formule (IV) que l'on fait réagir avec un composé de formule (V) : 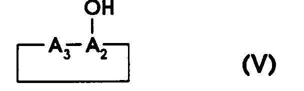 dans laquelle A₂ et A₃ ont la même signification que dans la formule (I),
(dérivé de formule (V) lui-même obtenu par couplage peptidique classique des amino-acides optiquement purs, A₃-OH et A₂-OH protégés, cyclisation en dicétopipérazine correspondante en milieu basique, déprotection et purification), en présence d'un réactif de couplage classique de la synthèse peptidique décrit précédemment, pour conduire à un composé de formule (I/a): 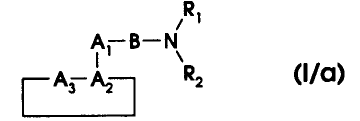 dans laquelle A₁, A₂, A₃, B, R₁ et R₂ ont la même signification que dans la formule (I),
le radical A₁, lorsqu'il représente un résidu tryptophane pouvant être éventuellement protégé par un réactif approprié, contenant le radical Q, par exemple Q-Cl, en présence de réactifs et de solvants convenables dans ce cas, tels que hydroxyde de sodium et hydrogénosulfate de tétrabutylammonium dans le chlorure de méthylène,
composé de formule (I/a) dont le résidu tryptophane est éventuellement protégé : que l'on purifie par une technique classique de purification,
que l'on transforme, le cas échéant, en ses sels d'addition à un acide ou à une base pharmaceutiquement acceptable,
qui, lorsqu'il contient une liaison pseudopeptique -CH₂-NH- ou -CH₂-S- sont préparés selon le procédé décrit précédemment,
l'introduction de la liaison -CH₂-NH- se faisant en préparant en solution l'aldéhyde P₁-NH-CHR-CHO (P₁ = groupement protecteur choisi parmi Boc, Fmoc, Z) selon la technique décrite par FEHRENTZ et CASTRO (Synthesis, 676-678, 1983) et en le condensant avec la chaîne peptidique croissante soit sur phase solide, selon la technique décrite par SASAKI et Coy (Peptides, 8, 119-121, 1988), soit en solution, l'introduction de la liaison -CH₂-S- se faisant en condensant le thiol de formule P₁-NH-CHR-CH₂SH, préparé à partir de l'alcool correspondant, sur le bromure de désamino-peptide C-terminal.

Les composés de l'invention possèdent des propriétés pharmacologiques très intéressantes. Ce sont des ligands spécifiques aux récepteurs des tachykinines qui possèdent des propriétés antagonistes vis-à-vis des récepteurs NK₁, NK₂ et NK₃. Ces propriétés antagonistes sont particulièrement intenses vis-à-vis des récepteurs NK₁. Les récepteurs NK₁ et NK₃ sont impliqués dans la régulation de la transmission de la douleur et l'augmentation de la perméabilité vasculaire. De plus, la stimulation des récepteurs NK₁ induit une hypersalivation, celle des récepteurs NK₂ entraîne tachycardie, hypotension et bronchoconstriction, enfin celle des récepteurs NK₃ est suivie de bradycardie, d'hypotension et de vasodilatation périphérique (D. REGOLI et coll., TIPS, 9, 290-295, 1988).

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, crèmes, pommades, gels dermiques, les aérosols.

La posologie varie selon l'âge et le poids du patient, la nature et la sévérité de l'affection ainsi que la voie d'administration.

Celle-ci peut être orale, nasale, rectale ou parentérale. D'une manière générale, elle s'échelonne entre 0,2 et 100 mg pour un traitement en une ou plusieurs prises par 24 heures.

Les exemples suivants illustrent l'invention et ne la limitent en aucune façon.

Les acides aminés dont les abréviations commencent par une lettre majuscule sont de configuration L.
Les acides aminés dont les abréviations commencent par une lettre minuscule sont de configuration D.
La lettre ψ indique la présence d'une liaison pseudopeptidique dont la nature est indiquée entre parenthèses.

Les préparations suivantes ne permettent pas d'obtenir les composés de l'invention mais conduisent à des intermédiaires utiles dans la préparation des composés de l'invention.

### PREPARATION A:

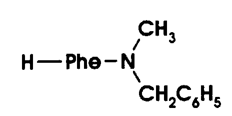

CF₃CO₂H

60 mmoles de Boc-Phe-OH et 60 mmoles de méthylbenzylamine sont dissoutes dans 150 ml de diméthylformamide. 60 mmoles d'hydroxy-benzotriazole (HOBT) dans 100 ml de diméthylformamide sont ajoutées au mélange précédent puis 80 mmoles de dicyclohexylcarbodiimide (DCC) en solution dans 40 ml de diméthylformamide. L'agitation est maintenue 16 heures à température ambiante.

Après filtration de la dicyclohexylurée formée et évaporation du solvant, le résidu est repris par de l'acétate d'éthyle et la phase organique est lavée par une solution à 5 % d'hydrogénocarbonate de sodium puis par une solution de sulfate de potassium et enfin par une solution saturée de chlorure de sodium.

Après évaporation du solvant, le résidu brut est purifié par chromatographie sur colonne de silice. La fonction amine terminale de la phénylalanine est déprotégée par traitement du composé obtenu pendant 20 minutes dans de l'acide trifluoroacétique, ou par l'acide chlorhydrique gazeux dans l'acétate d'éthyle. Le produit attendu est alors obtenu sous forme de sel.
Rendement : 92 %

### PREPARATION B:

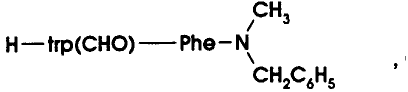

CF₃CO₂H

50 mmoles du composé obtenu dans la préparation A sont dissoutes dans 100 ml de diméthylformamide en présence de 50 mmoles de triéthylamine. Une solution contenant 50 mmoles de Boc-trp (CHO)-OH, 50 mmoles de HOBT dans 70 ml de diméthylformamide est ajoutée au mélange précédent, suivie par l'addition d'une solution contenant 70 mmoles de DCC dans 30 ml de diméthylformamide. L'ensemble est maintenu 16 heures sous agitation à température ambiante. Le produit attendu sous forme de trifluoroacétate est isolé, purifié et déprotégé procédant comme dans la préparation A.
Rendement : 78 %

### EXEMPLE 1:

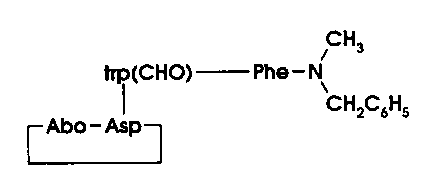

### STADE A : Fmoc-Asp(OtBu)-Abo-OMe

35 mmoles de H-Abo-OMe, HCI sont dissoutes dans 70 ml de diméthylformamide en présence de 35 mmoles de triéthylamine. Une solution contenant 35 mmoles de Fmoc-Asp(OtBu)-OH et 35 mmoles de HOBT est alors ajouté au mélange précédent, suivie par l'addition d'une solution contenant 42 mmoles de DCC dans 20 ml de diméthylformamide. L'ensemble est maintenu 14 heures sous agitation à température ambiante. Le produit attendu est isolé en procédant comme dans la préparation A et purifié par chromatographie sur colonne de silice en utilisant comme solvant d'élution un mélange acétate d'éthyle/pentane : 1/1.
Rendement : 96 %

### STADE B:

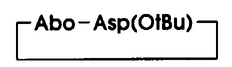

12 mmoles du composé obtenu au stade précédent sont dissoutes dans 60 ml d'un mélange diméthylformamide/pipéridine (80/20). L'ensemble est maintenu sous agitation pendant 15 minutes à température ambiante.

Après refroidissement et évaporation du solvant, le produit cyclisé attendu est obtenu et purifié sur colonne de silice en utilisant l'acétate d'éthyle comme solvant d'élution.
Rendement : 69 %

### STADE C :

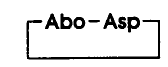

9 mmoles du composé obtenu au stade précédent sont dissoutes dans 80 ml d'un mélange dichlorométhane/acide acétique (50/50) et l'ensemble est agité 1 heure à température ambiante.

Après évaporation des solvants, le résidu est repris par de l'éther éthylique. Le produit attendu précipite, est isolé par filtration, puis lavé et séché.
Rendement : 69 %

### STADE D:

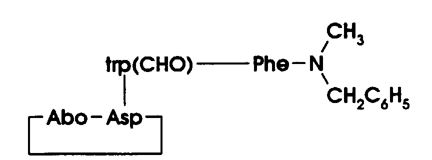

1,7 mmoles du composé obtenu dans la préparation B sont dissoutes dans 15 ml de diméthylformamide en présence de 1,7 mmoles de triéthylamine. Une solution contenant 1,7 mmoles du composé obtenu au stade C et 1,7 mmoles de HOBT dans 12 ml de diméthylformamide est ajoutée au mélange précédent, suivie par l'addition d'une solution contenant 1,8 mmoles de DCC dans 1 ml de diméthylformamide. L'ensemble est maintenu 14 heures sous agitation à température ambiante. Le produit attendu est isolé en procédant comme dans la préparation A et purifié par chromatographie sur silice C₁₈ en utilisant comme solvant d'élution un mélange acétonitrile/eau/acide trifluoroacétique (40/60/0,2).
Rendement : 64 %
Spectre de masse (FAB) MH⁺ : m/_{z} = 717 (masse moléculaire : 716,8)

L'analyse du produit obtenu est réalisée après décomposition de celui-ci en acides aminés par hydrolyse acide et dosage quantitatif des acides aminés obtenus par chromatographie liquide :

| | **Abo** | **Asp** | **Phe +Trp** |
|---|---|---|---|
| **% calculé** | 1 | 1 | 2 |
| **% trouvé** | 1,09 | 1,09 | 1,82 |

Les exemples suivants ont été obtenus en utilisant le même procédé de synthèse que celui décrit dans l'exemple 1.

### EXEMPLE 2:

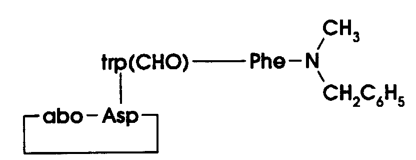

Spectre de masse (Fab) : MH⁺ : m/_{z} = 717 (masse moléculaire : 716,8)

### EXEMPLE 3 :

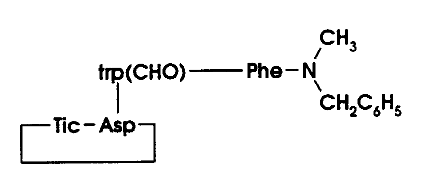

Spectre de masse (FAB) : MH⁺ : m/_{z} = 739 (masse moléculaire : 738,8)

### EXEMPLE 4:

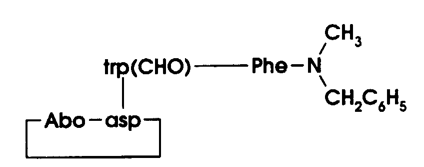

Spectre de masse (FAB) : MH⁺ : m/_{z} = 717 (masse moléculaire : 716,8)

### EXEMPLE 5 :

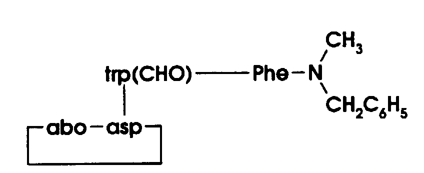

Spectre de masse (FAB) : MH⁺ : m/_{z} = 717 (masse moléculaire : 716,8)

### EXEMPLE 6 :

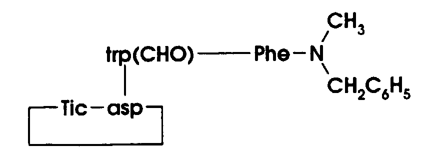

Spectre de masse (FAB) : MH⁺ : m/_{z} = 739 (masse moléculaire : 738,8)

### EXEMPLE 7 :

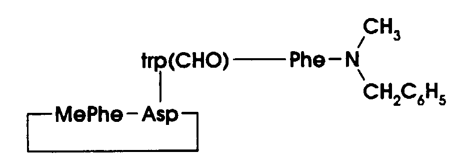

Spectre de masse (FAB) : MH⁺ : m/_{z} = 741 (masse moléculaire : 740,8)

### EXEMPLE 8 :

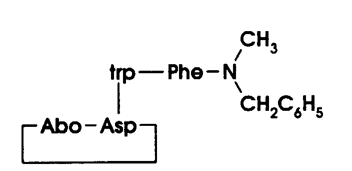

Spectre de masse (FAB) : MH⁺ : m/z = 689 (masse moléculaire : 688,8)

### EXEMPLE 9 :

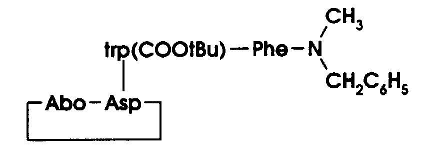

Composé obtenu à partir du composé de l'exemple 8, la protection du tryptophane étant réalisé par (Boc)₂O en présence de triméthyl-aminopyridine.
Spectre de masse (FAB) : MH⁺ : m/_{z} : 789 (masse moléculaire : 788)

### EXEMPLE 10:

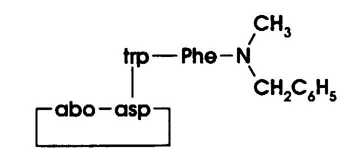

Spectre de masse (FAB) : MH⁺ : m/_{z} = 689 (masse moléculaire : 688,8)

Dans les exemples suivants, on procède comme dans l'exemple 1 mais on utilise au stade D le produit obtenu dans la préparation A.

### EXEMPLE 11:

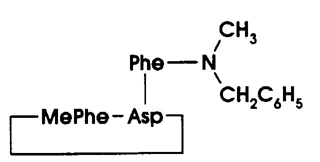

Spectre de masse (FAB) : MH⁺ : m/_{z} = 527 (masse moléculaire : 526,6)

### EXEMPLE 12:

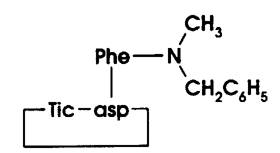

Spectre de masse (FAB) : MH⁺ : m/_{z} = 525 (masse moléculaire : 524,6)

### EXEMPLE 13:

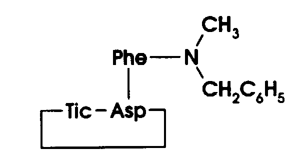

Spectre de masse (FAB) : MH⁺ : m/_{z} = 525 (masse moléculaire : 524,6)

### EXEMPLE 14:

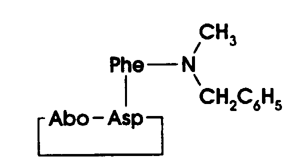

Spectre de masse (FAB) : MH⁺ : m/_{z} = 503 (masse moléculaire : 502,6)

### EXEMPLE 15 :

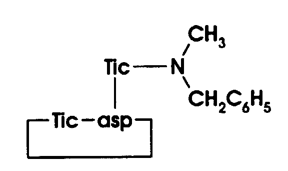

Composé obtenu en remplaçant, dans la préparation A, Boc-Phe-OH par Boc-Tic-OH.

### EXEMPLE 16:

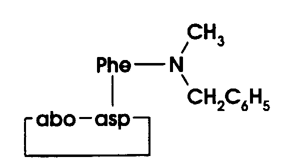

Spectre de masse (FAB) : MH⁺ : m/_{z} = 503 (masse moléculaire : 502,6)

### EXEMPLE 17:

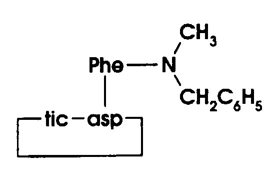

### EXEMPLE 18:

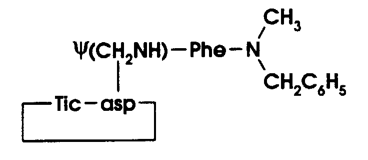

### EXEMPLE 19:

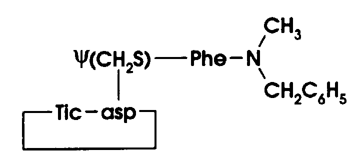

Les exemples suivants ont été réalisés selon l'exemple 1 :

### EXEMPLE 20:

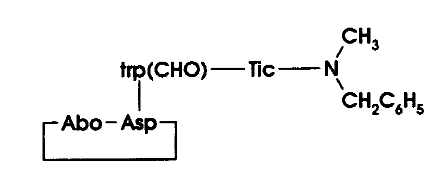

Spectre de masse (FAB) : MH⁺ : m/_{z} = 729 (masse moléculaire : 728,8)

### EXEMPLE 21 :

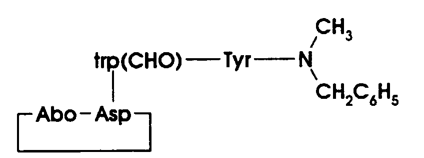

Spectre de masse (FAB) : MH⁺ : m/_{z} = 733 (masse moléculaire : 732,8)

### EXEMPLE 22 :

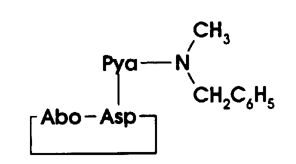

Spectre de masse (FAB) : MH⁺ : m/_{z} = 504 (masse moléculaire : 503,6)

### EXEMPLE 23 :

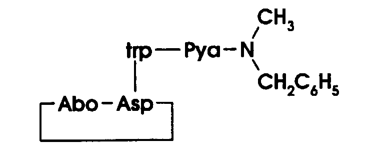

Spectre de masse (FAB) : MH⁺ : m/_{z} = 690 (masse moléculaire : 689,8)

### EXEMPLE 24:

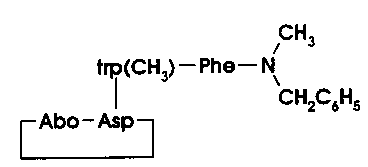

Spectre de masse (FAB) : MH⁺ : m/_{z} = 703 (masse moléculaire : 702)

### EXEMPLE 25:

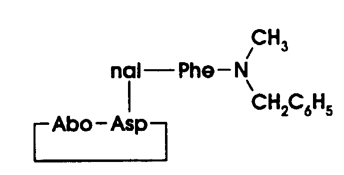

Composé synthétisé de manière identique à l'exemple 1, le composé de la préparation B étant obtenu à partir de Boc-nal-OH.
Spectre de masse (FAB) : MH⁺ : m/_{z} = 700 (masse moléculaire : 699,85)

### EXEMPLE 26 :

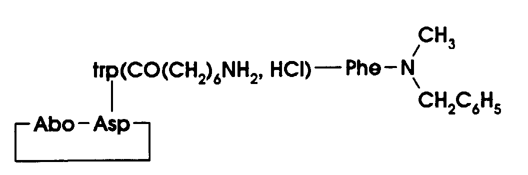

### STADE A : Boc-NH-(CH₂)₆-COOH

5 g (34,4 mmoles) d'acide 7-amino-heptanoïque sont dissous dans 80 ml d'un mélange dioxanne/eau (45/35). 34,4 ml de soude 1N sont ajoutés à 0°C. 8,27 g (37,84 mmoles) de ditert-butyl dicarbonate préalablement dissous dans 25 ml de dioxanne sont ajoutés goutte à goutte.
Après une nuit sous agitation et acidification à pH = 2 par une solution d'hydrogénosulfate de potassium à 15 %, la phase organique, après traitement habituel fournit un gel jaune, précipitant sous forme de poudre blanche (8,40 g) dans un mélange éther/pentane.
Rendement : 100 %

### STADE B : Boc-NH-(CH₂)₆-COO-Np (Np représentant le groupement paranitrophényle)

A 3 g (12,2 mmoles) de composé obtenu au stade A dans 10 ml de dichlorométhane sont ajoutés 1,874 g (13,4 mmoles) de paranitrophénol, puis 2,775 g (13,4 mmol) de DCC. Après deux jours sous agitation à l'abri de la lumière, l'urée formée au cours de la réaction est éliminée par filtration. Le traitement par le pentane de la phase organique concentrée, fournit 3,23 g d'un précipité jaune pâle.
Rendement : 72 %

### STADE C :

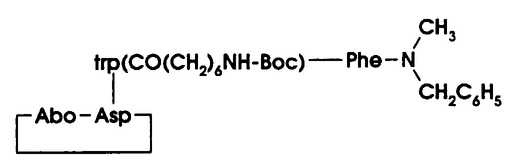

A 0,959 g (3,63 mmoles) d'éther-couronne 18-6 dans 40 ml de tétrahydrofurane sont ajoutés 2,5 g (3,63 mmoles) de composé obtenu dans l'exemple 33, 1,662 g (4,54 mmoles) du composé obtenu au stade B et 791 µl (4,54 mmoles) de diisopropyléthylamine et 422 mg de fluorure de potassium.
Le mélange est abandonné 70 heures sous agitation à l'abri de la lumière puis est concentré sous vide. Le milieu réactionnel est repris par un mélange éther/acétate d'éthyle, puis, après traitement habituel de la phase organique et reprise dans l'éther, un produit blanc-cassé précipite.
Celui-ci est purifié par chromatographie sur silice (éluant chloroforme/méthanol 97/3) pour donner 1,59 g de produit attendu sous forme cristalline blanche.
Rendement : 47 %

### STADE D :

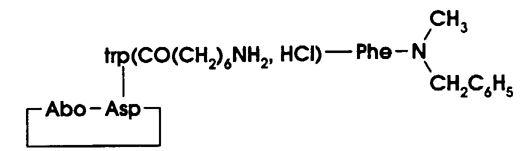

1,5 g (1,64 mmoles) de peptide protégé obtenu au stade C sont dissous dans 100 ml d'une solution d'acétate d'éthyle saturée en acide chlorhydrique gazeux. Après 1 h 30 sous agitation à température ambiante, évaporation du solvant, précipitation dans l'éther et purification par HPLC préparative sur phase inverse, puis sur colonne de résine échangeuse d'anions (Amberlite IRA-93), et reformation du chlorhydrate : 723 mg de poudre blanc-cassé correspondante au produit attendu sont obtenus.
Rendement : 52 %

Les deux exemples suivants ont été réalisés en utilisant le même procédé de synthèse que celui décrit pour l'exemple 26.

### EXEMPLE 27:

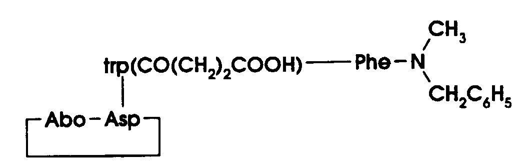

Spectre de masse (FAB) : MH⁺ : m/_{z} = 789 (masse moléculaire : 788,9)

### EXEMPLE 28:

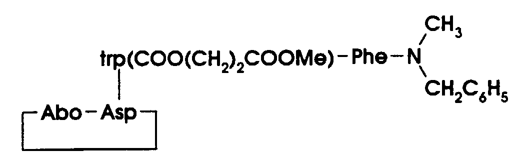

Spectre de masse (FAB) : MH⁺ : m/_{z} = 803 (masse moléculaire : 802,9)

Les exemples 29 et 30 ont été préparés comme l'exemple 26, avec les modifications suivantes :
- solvant CH₂Cl₂ (au lieu du THF),
- NaOH broyée (au lieu de l'éther-couronne et de fluorure de potassium),
- agent acylant : chlorure d'acide (au lieu d'ester actif ou anhydride), en présence de tétrabutylammonium bisulfate, comme catalyseur de transfert de phase.

### EXEMPLE 29:

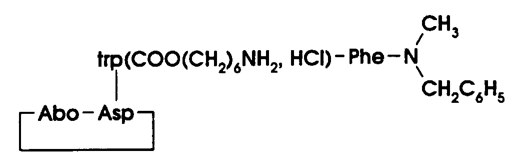

Spectre de masse (FAB) : MH⁺ : m/_{z} = 832 (masse moléculaire, base libre : 832)

### EXEMPLE 30:

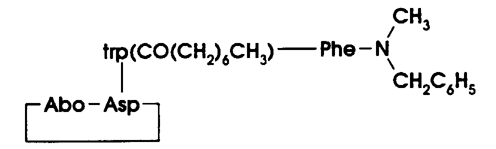

Le composé obtenu dans l'exemple 8 en solution dans le chlorure de méthylène est additionné de chlorure de n-octanoyle, d'hydroxyde de sodium finement pulvérisé et d'hydrogénosulfate de tétrabutylammonium, pour conduire au produit attendu.
Spectre de masse (FAB) : MH⁺ : m/_{z} = 815 (masse moléculaire : 815)

Les exemples suivants ont été réalisés en utilisant le même procédé de synthèse que celui décrit pour l'exemple 30.

### EXEMPLE 31 :

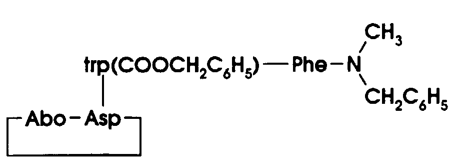

Spectre de masse (FAB) : MH⁺ : m/_{z} = 823 (masse moléculaire : 822)

### EXEMPLE 32 :

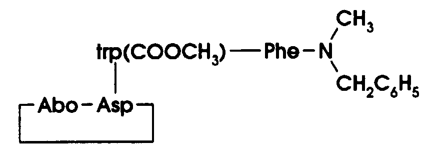

Spectre de masse (FAB) : MH⁺ : m/_{z} = 823 (masse moléculaire : 822)

### EXEMPLE 33 :

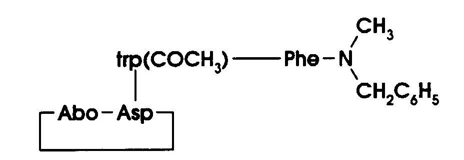

Spectre de masse (FAB) : MH⁺ : m/_{z} = 731 (masse moléculaire : 730)

### EXEMPLE 34 :

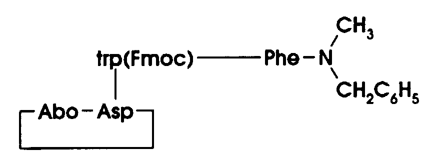

Spectre de masse (FAB) : MH⁺ : m/_{z} = 911 (masse moléculaire : 911,1)

### ETUDE PHARMACOLOGIQUE DES DERIVES DE L'INVENTION

### EXEMPLE 35 : Bio-essais sur le muscle lisse isolé

Afin d'évaluer le potentiel antagoniste des neurokinines des composés de l'invention, trois préparations de muscle lisse ont été utilisées. Chacune de ces préparations, décrite comme présentant une bonne spécificité pour un type de récepteur aux neurokinines, a été réalisée conformément aux techniques suivantes décrites dans la littérature :
- veine cave de lapin pour l'étude du récepteur NK₁ selon D.REGOLI et Coll. (J. Cardiovasc. Pharmacol., sous presse) ;
- artère pulmonaire de lapin sans endothélium pour l'étude de récepteur NK₂ selon D.REGOLI et Coll. (European J. Pharmacol. 125, 37-44, 1985) ;
- veine porte de rat pour l'étude du récepteur NK₃ selon D.REGOLI et Coll. (European J. Pharmacol. 134, 321-326, 1986).

Le pouvoir antagoniste des composés de l'invention est exprimé sous forme de pA₂ tel que défini par O.ARUNLAKSHANA et H.O.SCHILD (Brit. J. Pharmacol., 14, 48-58, 1959). Les résultats à titre d'exemples limitatifs sont regroupés dans le tableau ci-dessous.

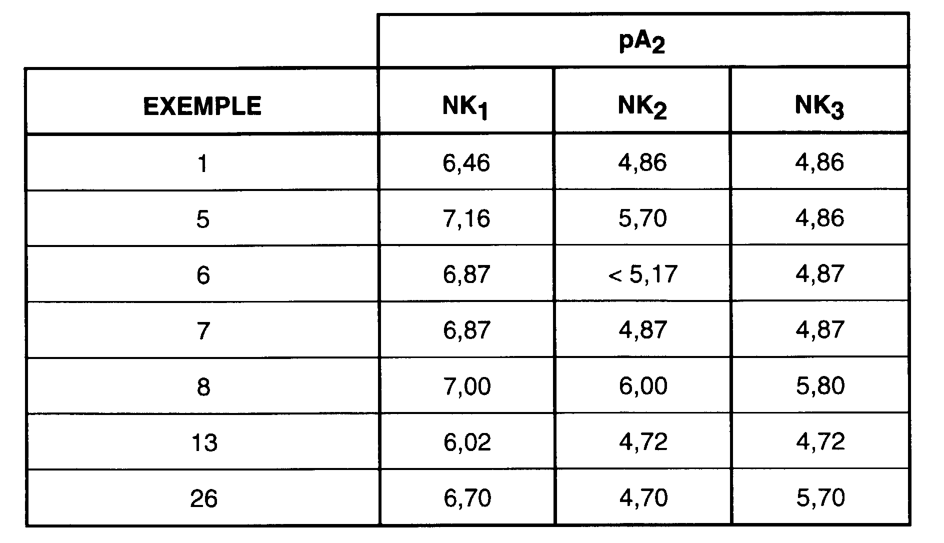

Les composés de l'invention présentent une puissante activité antagoniste vis-à-vis des récepteurs NK₁, avec, pour la plupart, une moindre activité pour les récepteurs NK₂ et NK₃.

### EXEMPLE 36 : Activité in vivo. Test d'Eddy chez la souris.

En raison de l'implication de la substance P dans la transmission de la douleur au niveau spinal (M. OTSUKA et S. KONISHI, TINS, 6, 317-320, 1983), l'activité pharmacologique in vivo des composés de l'invention a été recherchée chez la souris sur le test d'hyperalgésie thermique initialement décrit par N.B. EDDY et Coll. (J. Pharmacol. Exp. Ther., 107, 385-393, 1953). Ce test consiste à mesurer le temps de réaction à la chaleur déterminé par le léchage des pattes antérieures chez une souris (CD₁ mâle, Ch. River, 25-30 g) placée sur une plaque métallique chauffée à 55°C.

Les animaux ont été traités avec les composés d'invention, 5 minutes avant le passage sur la plaque chauffante par voie intraveineuse.

La moyenne des temps de réaction obtenue pour chaque lot traité (12 souris par lot) a été comparée à la moyenne du lot témoin correspondant. Les résultats sont exprimés sous forme de DE₅₀ qui correspond à la dose augmentant de 50 % le temps de réaction. Ces résultats sont regroupés dans le tableau ci-dessous.

| **EXEMPLE** | **Test d'Eddy chez la souris DE**_{**50**} **(mg/kg IV)** |
|---|---|
| 1 | 0,2 |
| 5 | 3,5 |
| 6 | 3,0 |
| 7 | 4,0 |
| 8 | 0,5 |
| 13 | 3,0 |
| 26 | 2,0 |
| morphine | 0,5 |

Les composés de l'invention présentent des propriétés antalgiques importantes. Le composé de l'exemple 1 possède plus particulièrement un pouvoir analgésique supérieur à celui de la morphine.

L'activité antalgique des dérivés de l'invention qui mobiliserait tout autre récepteur que ceux connus des neurokinines est également revendiquée.

### COMPOSITION PHARMACEUTIQUE

### EXEMPLE 37 : Comprimé : formule de préparation pour 1000 comprimés dosés à 2 mg

| | |
|---|---|
| Composé de l'exemple 1 | 2 g |
| Hydroxypropyl cellulose | 2 g |
| Amidon de blé | 10 g |
| Lactose | 100 g |
| Stéarate de magnésium | 3 g |
| Talc | 3 g |

## Revendications (Revendications pour l'(les) Etat(s) contractant(s) suivant(s): AT, BE, CH, DE, DK, FR, GB IT, LI, LU, NL, PT, SE)

1. Composés de formule (I) : 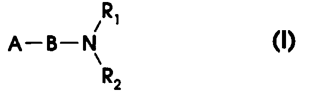 dans laquelle :
R₁ et R₂ identiques ou différents représentent un atome d'hydrogène, un groupement alkyle (C₁-C₆) linéaire ou ramifié, un groupement cycloalkyle (C₃-C₇) ou un groupement benzyle,
B représente un résidu d'amino-acide aromatique, choisi parmi phénylalanine (Phe), tyrosine (Tyr), (1,2,3,4)-tétrahydroisoquinoléine-3-carbonyl (Tic), tryptophane (Trp), tryptophane protégé par un radical formyle (Trp (CHO)) ou pyridinylalanine (Pya).
A représente un résidu peptidique de formule : 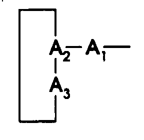
dans lequel :
A₁ représente une liaison, un résidu 2-azabicyclo[2.2.2]octane-3-carbonyl (Abo), leucine (Leu), β-naphtylalanine (Nal), tryptophane (Trp), tryptophane protégé par un radical Q (Trp(Q)),
Q représentant le radical
-X-(CH₂)n-R'
dans lequel
X représente une liaison, -CO-, ou -COO-,
n est un entier de 0 à 10,
R' est un atome d'hydrogène, un radical alkyle (C₁-C₁₀) linéaire ou ramifié, benzyle, 9-fluorénylméthyle, -NH₂, -COOH ou -COOR" (R" = alkyl (C₁-C₆) linéaire ou ramifié),
A₂ représente un résidu acide aspartique (Asp),
A₃ représente un résidu (1,2,3,4)-tétrahydroisoquinoléine-3-carbonyl (Tic), 2-azabicyclo[2.2.2]octane-3-carbonyl (Abo), ou méthylphénylalanine (MePhe),
étant entendu que la liaison peptidique (-CO-NH-) entre A₁ et A₂ ou entre A₂ et B dans le cas où A₁ est une liaison, peut être remplacée par une liaison pseudopeptidique choisie parmi -CH₂-NH- ou -CH₂-S-,
leurs énantiomères, diastéréoisomères et épimères ainsi que leurs sels d'addition à un acide ou à une base pharmaceutiquement acceptable,
étant entendu que chaque amino-acide de la séquence peptidique est optiquement pur et que le carbone α de chaque amino-acide peut être de configuration D ou L.

2. Composé selon la revendication 1 qui est le 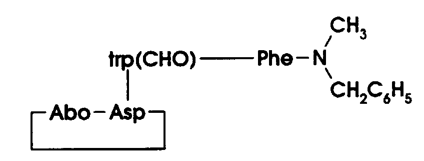 ses énantiomères, diastéréoisomères et épimères.

3. Composé selon la revendication 1 qui est le 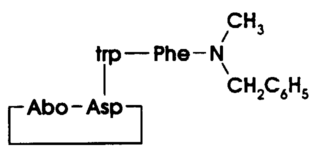 ses énantiomères, diastéréoisomères et épimères.

4. Composé selon la revendication 1 qui est le 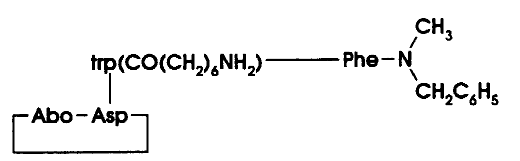 ses énantiomères, diastéréoisomères et épimères, ainsi que ses sels d'ammonium.

5. Composé selon la revendication 1 qui est le 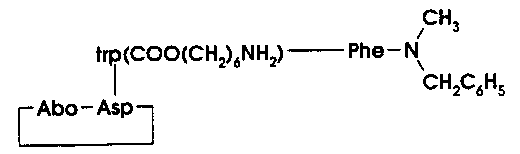 ses énantiomères, diastéréoisomères et épimères, ainsi que ses sels d'ammonium.

6. Procédé de préparation des composés de formule (I) selon la revendication 1 caractérisé en ce que l'on fait réagir un amide de formule (II), optiquement pur : 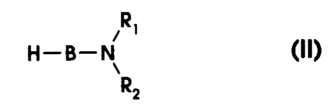 dans laquelle B, R₁ et R₂ ont la même signification que dans la formule (I), avec un deuxième amino-acide, optiquement pur, dont la fonction amine N-terminale est protégée, de formule (III) :
P₁-A₁-OH (III)
dans laquelle :
P₁ représente un groupement protecteur choisi parmi benzyl-oxycarbonyl (Z), tert-butyloxycarbonyl (Boc), 9-fluorényl- méthylcarbonyl (Fmoc),
A₁ a la même signification que dans la formule (I),
en présence d'un réactif de couplage classique de la synthèse peptidique choisi parmi le couple dicyclohexylcarbodiimide (DCC)-hydroxybenzo-triazole (HOBT), le benzotriazol-1-oxytris (diméthylamino) phosphonium hexafluorophosphate (BOP) ou encore le diphénylphosphorylazide (DPPA),
pour conduire, après déprotection sélective de la fonction amine N-terminale, au composé de formule (IV) : 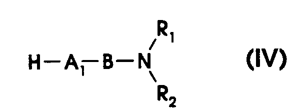 dans laquelle A₁, B, R₁ et R₂ ont les mêmes significations que dans la formule (I), composé de formule (IV) que l'on fait réagir avec un composé de formule (V) : 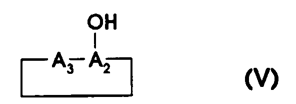 dans laquelle A₂ et A₃ ont la même signification que dans la formule (I),
(dérivé de formule (V) lui-même obtenu par couplage peptidique classique des amino-acides optiquement purs, A₃-OH et A₂-OH protégés, cyclisation en dicétopipérazine correspondante en milieu basique, déprotection et purification), en présence d'un réactif de couplage classique de la synthèse peptidique décrit précédemment, pour conduire à un composé de formule (I/a): 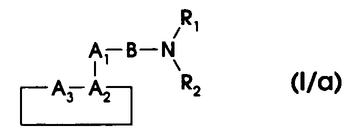 dans laquelle A₁, A₂, A₃, B, R₁ et R₂ ont la même signification que dans la formule (I),
le radical A₁, lorsqu'il représente un résidu tryptophane pouvant être éventuellement protégé par un réactif approprié, contenant le radical Q, défini dans la formule (I),
composé de formule (I/a) dont le résidu tryptophane est éventuellement protégé : que l'on purifie par une technique classique de purification,
que l'on transforme, le cas échéant, en leurs sels d'addition à un acide ou à une base pharmaceutiquement acceptable,
qui, lorsqu'ils contiennent une liaison pseudopeptique -CH₂-NH- ou -CH₂-S- sont préparés selon le procédé décrit précédemment,
l'introduction de la liaison -CH₂-NH-, se faisant en préparant en solution l'aldéhyde P₁-NH-CHR-CHO (P₁ = groupement protecteur choisi parmi Boc, Fmoc, Z) et en le condensant avec la chaîne peptidique croissante soit sur phase solide, soit en solution,
l'introduction de la liaison -CH₂-S- se faisant en condensant le thiol de formule P₁-NH-CHR-CH₂SH, préparé à partir de l'alcool correspondant, sur le bromure de désamino-peptide C-terminal.

7. Compositions pharmaceutiques contenant comme principe actif au moins un composé selon l'une quelconque des revendications 1 à 5, seul ou en combinaison avec un ou plusieurs excipients ou véhicules inertes, non toxiques, pharmaceutiquement acceptables.

8. Compositions pharmaceutiques selon la revendication 7 contenant au moins un principe actif selon l'une quelconque des revendications 1 à 5 utiles comme antagonistes de substance P dans le traitement de la douleur, de l'inflammation, des troubles gastro-intestinaux, de l'asthme, des allergies et des maladies du système nerveux central.

## Revendications (Revendications pour l'(les) Etat(s) contractant(s) suivant(s): GR, ES)

1. Procédé de préparation des composés de formule (I) : 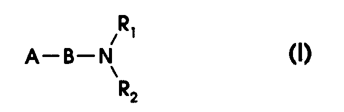 dans laquelle :
R₁ et R₂ identiques ou différents représentent un atome d'hydrogène, un groupement alkyle (C₁-C₆) linéaire ou ramifié, un groupement cycloalkyle (C₃-C₇) ou un groupement benzyle,
B représente un résidu d'amino-acide aromatique, choisi parmi phénylalanine (Phe), tyrosine (Tyr), (1,2,3,4)-tétrahydroisoquinoléine-3-carbonyl (Tic), tryptophane (Trp), tryptophane protégé par un radical formyle (Trp (CHO)) ou pyridinylalanine (Pya).
A représente un résidu peptidique de formule : 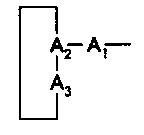
dans lequel :
A₁ représente une liaison, un résidu 2-azabicyclo[2.2.2]octane-3-carbonyl (Abo), leucine (Leu), β-naphtylalanine (Nal), tryptophane (Trp), tryptophane protégé par un radical Q (Trp(Q)),
Q représentant le radical
-X-(CH₂)n-R'
dans lequel
X représente une liaison, -CO-, ou -COO-,
n est un entier de 0 à 10,
R' est un atome d'hydrogène, un radical alkyle (C₁-C₁₀) linéaire ou ramifié, benzyle, 9-fluorénylméthyle, -NH₂, -COOH ou -COOR" (R" = alkyl (C₁-C₆) linéaire ou ramifié),
A₂ représente un résidu acide aspartique (Asp),
A₃ représente un résidu (1,2,3,4)-tétrahydroisoquinoléine-3-carbonyl (Tic), 2-azabicyclo[2.2.2]octane-3-carbonyl (Abo), ou méthylphénylalanine (MePhe),
étant entendu que la liaison peptidique (-CO-NH-) entre A₁ et A₂ ou entre A₂ et B dans le cas où A₁ est une liaison, peut être remplacée par une liaison pseudopeptidique choisie parmi -CH₂-NH- ou -CH₂-S-,
leurs énantiomères, diastéréoisomères et épimères ainsi que leurs sels d'addition à un acide ou à une base pharmaceutiquement acceptable,
étant entendu que chaque amino-acide de la séquence peptidique est optiquement pur et que le carbone α de chaque amino-acide peut être de configuration D ou L, caractérisé en ce que l'on fait réaqir un amide de formule (II), optiquement pur : 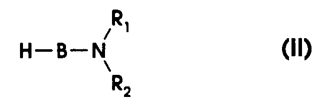 dans laquelle B, R₁ et R₂ ont la même signification que dans la formule (I), avec un deuxième amino-acide, optiquement pur, dont la fonction amine N-terminale est protégée, de formule (III):
P₁-A₁-OH (III)
dans laquelle :
P₁ représente un groupement protecteur choisi parmi benzyl-oxycarbonyl (Z), tert-butyloxycarbonyl (Boc), 9-fluorényl- méthylcarbonyl (Fmoc),
A₁ a la même signification que dans la formule (I),
en présence d'un réactif de couplage classique de la synthèse peptidique choisi parmi le couple dicyclohexylcarbodiimide (DCC)-hydroxybenzo-triazole (HOBT), le benzotriazol-1-oxytris (diméthylamino) phosphonium hexafluorophosphate (BOP) ou encore le diphénylphosphorylazide (DPPA),
pour conduire, après déprotection sélective de la fonction amine N-terminale, au composé de formule (IV) : 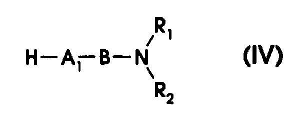 dans laquelle A₁, B, R₁ et R₂ ont les mêmes significations que dans la formule (I),
composé de formule (IV) que l'on fait réagir avec un composé de formule (V) : 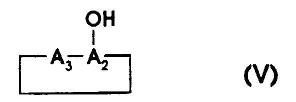 dans laquelle A₂ et A₃ ont la même signification que dans la formule (I),
(dérivé de formule (V) lui-même obtenu par couplage peptidique classique des amino-acides optiquement purs, A₃-OH et A₂-OH protégés, cyclisation en dicétopipérazine correspondante en milieu basique, déprotection et purification), en présence d'un réactif de couplage classique de la synthèse peptidique décrit précédemment, pour conduire à un composé de formule (I/a) : 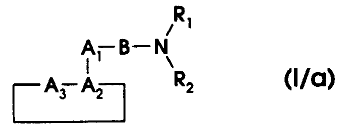 dans laquelle A₁, A₂, A₃, B, R₁ et R₂ ont la même signification que dans la formule (I),
le radical A₁, lorsqu'il représente un résidu tryptophane pouvant être éventuellement protégé par un réactif approprié, contenant le radical Q, défini dans la formule (I),
composé de formule (I/a) dont le résidu tryptophane est éventuellement protégé :
que l'on purifie par une technique classique de purification,
que l'on transforme, le cas échéant, en leurs sels d'addition à un acide ou à une base pharmaceutiquement acceptable,
qui, lorsqu'ils contiennent une liaison pseudopeptique -CH₂-NH- ou -CH₂-S- sont préparés selon le procédé décrit précédemment,
l'introduction de la liaison -CH₂-NH-, se faisant en préparant en solution l'aldéhyde P₁-NH-CHR-CHO (P₁ = groupement protecteur choisi parmi Boc, Fmoc, Z) et en le condensant avec la chaîne peptidique croissante soit sur phase solide, soit en solution,
l'introduction de la liaison -CH₂-S- se faisant en condensant le thiol de formule P₁-NH-CHR-CH₂SH, préparé à partir de l'alcool correspondant, sur le bromure de désamino-peptide C-terminal.

2. Procédé de préparation du composé selon la revendication 1 qui est le 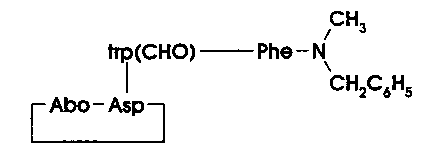 ses énantiomères, diastéréoisomères et épimères.

3. Procédé de préparation du composé selon la revendication 1 qui est le 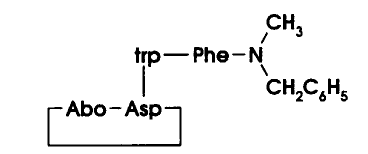 ses énantiomères, diastéréoisomères et épimères.

4. Procédé de préparation du composé selon la revendication 1 qui est le 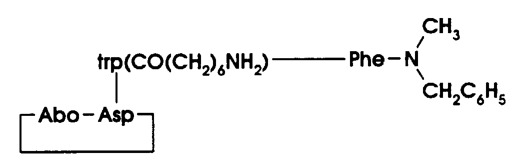 ses énantiomères, diastéréoisomères et épimères, ainsi que ses sels d'ammonium.

5. Procédé de préparation du composé selon la revendication 1 qui est le 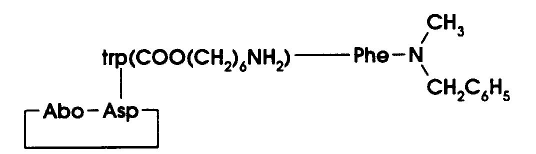 ses énantiomères, diastéréoisomères et épimères, ainsi que ses sels d'ammonium.

6. Procédé de préparation de compositions pharmaceutiques contenant comme principe actif au moins un composé selon l'une quelconque des revendications 1 à 5, seul ou en combinaison avec un ou plusieurs excipients ou véhicules inertes, non toxiques, pharmaceutiquement acceptables.

7. Procédé de préparation de compositions pharmaceutiques selon la revendication 6 contenant au moins un principe actif selon l'une quelconque des revendications 1 à 5 utiles comme antagonistes de substance P dans le traitement de la douleur, de l'inflammation, des troubles gastro-intestinaux, de l'asthme, des allergies et des maladies du système nerveux central.

## Claims (Claims for the following Contracting State(s): AT, BE, CH, DE, DK, FR, GB, IT, LI, LU, NL, PT, SE)

1. Compounds of formula (I) 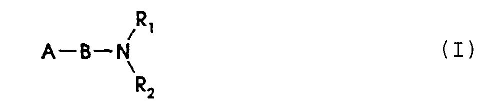 wherein :
R₁ and R₂, which are identical or different, each represents a hydrogen atom, a straight-chain or branched (C₁-C₆)-alkyl group, a (C₃-C₇)-cycloalkyl group or a benzyl group,
B represents an aromatic amino acid residue selected from phenylalanine (Phe), tyrosine (Tyr), (1,2,3,4)-tetrahydroisoquinoline-3-carbonyl (Tic), tryptophan (Trp), tryptophan protected by a formyl radical (trp (CHO)) and pyridinylalanine (Pya),
A represents a peptide residue of formula : 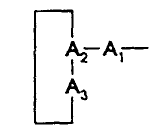
wherein :
A₁ represents a bond, a 2-azabicyclo[2.2.2]octane-3-carbonyl residue (Abo), a leucine residue (Leu), a β-naphthylalanine residue (Nal), a tryptophan residue (Trp), a tryptophan residue protected by a radical Q (Trp(Q)),
Q representing the radical
-X-(CH₂)ₙ-R'
wherein
X represents a bond, -CO- or -COO-,
n is an integer of from 0 to 10,
R' represents a hydrogen atom, a straight-chain or branched (C₁-C₁₀)-alkyl radical, a benzyl radical, a 9-fluorenylmethyl radical, -NH₂, -COOH or -COOR" (R" = straight-chain or branched (C₁-C₆)-alkyl)
A₂ represents an aspartic acid residue (Asp),
A₃ represents a (1,2,3,4)-tetrahydroisoquinoline-3-carbonyl residue (Tic), a 2-azabicyclo[2.2.2]octane-3-carbonyl residue (Abo) or a methylphenylalanine residue (MePhe),
it being understood that the peptide bond (-CO-NH-) between A₁ and A₂ or between A₂ and B in the case where A₁ is a bond may be replaced by a pseudopeptide bond selected from -CH₂-NH- and -CH₂-S-,
the enantiomers, diastereoisomers and epimers thereof and also the addition salts thereof with a pharmaceutically acceptable acid or base,
it being understood that each amino acid of the peptide sequence is optically pure and that the α-carbon atom of each amino acid may be in the D or L configuration.

2. Compound according to claim 1 which is 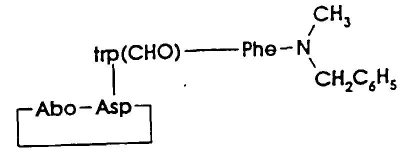 the enantiomers, diastereoisomers and epimers thereof.

3. Compound according to claim 1 which is 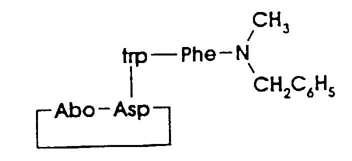 the enantiomers, diastereoisomers and epimers thereof.

4. Compound according to claim 1 which is 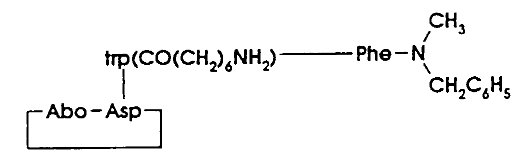 the enantiomers, diastereoisomers and epimers thereof and also the ammonium salts thereof.

5. Compound according to claim 1 which is 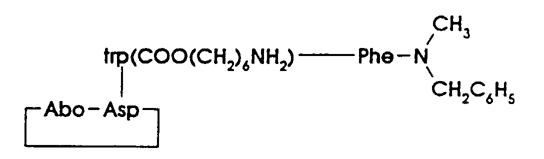 the enantiomers, diastereoisomers and epimers thereof and also the ammonium salts thereof.

6. Process for the preparation of compounds of formula (I) according to claim 1, characterised in that an optically pure amide of formula (II) : 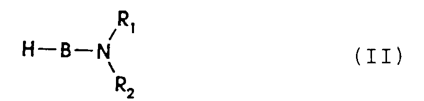 wherein B, R₁ and R₂ are as defined for formula (I), is reacted with a second optically pure amino acid, of which the N-terminal function is protected, of formula (III) :
P₁-A₁-OH (III)
wherein :
P₁ represents a protecting group selected from benzyloxycarbonyl (Z), tert-butyloxycarbonyl (Boc) and 9-fluorenyl-methylcarbonyl (Fmoc),
A₁ is as defined for formula (I),
in the presence of a conventional coupling reagent for peptide synthesis selected from the dicyclohexylcarbodiimide (DCC)-hydroxybenzotriazole (HOBT) couple, benzotriazol-1-oxytris(dimethylamino)phosphonium hexafluorophosphate (BOP) and also diphenylphosphoryl azide (DPPA), to yield, after selective deprotection of the N-terminal amine function, the compound of formula (IV) : 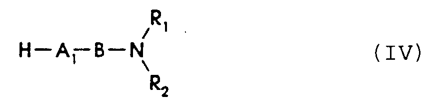 wherein A₁, B, R₁ and R₂ are as defined for formula (I),
which compound of formula (IV) is reacted with a compound of formula (V) : 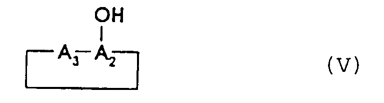 wherein A₂ and A₃ are as defined for formula (I), (the compound of formula (V) itself being obtained by conventional peptide coupling of protected optically pure amino acids A₃-OH and A₂-OH, cyclisation to form the corresponding diketopiperazine in basic medium, deprotection and purification), in the presence of a conventional coupling reagent for peptide synthesis described above, to yield a compound of formula (I/a) : 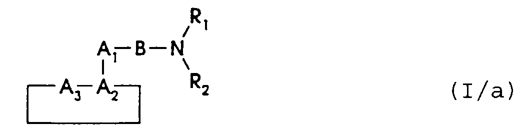 wherein A₁, A₂, A₃, B, R₁ and R₂ are as defined for formula (I),
it being possible for the radical A₁, when it represents a tryptophan residue, optionally to be protected by an appropriate reagent that contains the radical Q defined for formula (I),
which compound of formula (I/a), the tryptophan residue of which is optionally protected :
is purified by a conventional purification technique, is converted, if desired, into its addition salts with a pharmaceutically acceptable acid or base,
which when they contain a psuedopeptide bond -CH₂-NH- or -CH₂-S- are prepared according to the process described above,
the introduction of the bond -CH₂-NH- being effected by preparing in solution the aldehyde P₁-NH-CHR-CHO (P₁ = a protecting group selected from Boc, Fmoc, Z) and condensing the aldehyde with the growing peptide chain either in solid phase or in solution,
the introduction of the bond -CH₂-S- being effected by condensing the thiol of formula P₁-NH-CHR-CH₂SH, prepared from the corresponding alcohol, with the C-terminal desamino-peptide bromide.

7. Pharmaceutical compositions comprising as active ingredient at least one compound according to any one of claims 1 to 5, alone or in combination with one or more pharmaceutically acceptable, inert, non-toxic excipients or carriers.

8. Pharmaceutical compositions according to claim 7 comprising at least one active ingredient according to any one of claims 1 to 5 for use as antagonists of substance P in the treatment of pain, inflammation, gastrointestinal disorders, asthma, allergies, and disorders of the central nervous system.

## Claims (Claims for the following Contracting State(s): GR, ES)

1. Process for the preparation of compounds of formula (I) 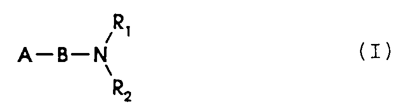 wherein :
R₁ and R₂, which are identical or different, each represents a hydrogen atom, a straight-chain or branched (C₁-C₆)-alkyl group, a (C₃-C₇)-cycloalkyl group or a benzyl group,
B represents an aromatic amino acid residue selected from phenylalanine (Phe), tyrosine (Tyr), (1,2,3,4)-tetrahydroisoquinoline-3-carbonyl (Tic), tryptophan (Trp), tryptophan protected by a formyl radical (trp (CHO)) and pyridinylalanine (Pya),
A represents a peptide residue of formula : 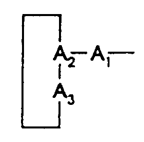 wherein :
A₁ represents a bond, a 2-azabicyclo[2.2.2]octane-3-carbonyl residue (Abo), a leucine residue (Leu), a β-naphthylalanine residue (Nal), a tryptophan residue (Trp), a tryptophan residue protected by a radical Q (Trp(Q)),
Q representing the radical
-X-(CH₂)ₙ-R'
wherein
X represents a bond, -CO- or -COO-,
n is an integer of from 0 to 10,
R' represents a hydrogen atom, a straight-chain or branched (C₁-C₁₀)-alkyl radical, a benzyl radical, a 9-fluorenylmethyl radical, -NH₂, -COOH or -COOR" (R" = straight-chain or branched (C₁-C₆)-alkyl)
A₂ represents an aspartic acid residue (Asp),
A₃ represents a (1,2,3,4)-tetrahydroisoquinoline-3-carbonyl residue (Tic), a 2-azabicyclo[2.2.2]octane-3-carbonyl residue (Abo) or a methylphenylalanine residue (MePhe),
it being understood that the peptide bond (-CO-NH-) between A₁ and A₂ or between A₂ and B in the case where A₁ is a bond may be replaced by a pseudopeptide bond selected from -CH₂-NH- and -CH₂-S-,
the enantiomers, diastereoisomers and epimers thereof and also the addition salts thereof with a pharmaceutically acceptable acid or base,
it being understood that each amino acid of the peptide sequence is optically pure and that the α-carbon atom of each amino acid may be in the D or L configuration, which process is characterised in that an optically pure amide of formula (II) : 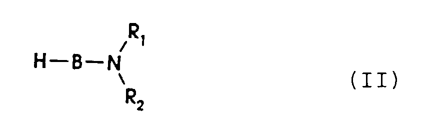 wherein B, R₁ and R₂ are as defined for formula (I), is reacted with a second optically pure amino acid, of which the N-terminal function is protected, of formula (III) :
P₁-A₁-OH (III)
wherein :
P₁ represents a protecting group selected from benzyloxycarbonyl (Z), tert-butyloxycarbonyl (Boc) and 9-fluorenyl-methylcarbonyl (Fmoc),
A₁ is as defined for formula (I),
in the presence of a conventional coupling reagent for peptide synthesis selected from the dicyclohexylcarbodiimide (DCC)-hydroxybenzotriazole (HOBT) couple, benzotriazol-1-oxytris(dimethylamino)phosphonium hexafluorophosphate (BOP) and also diphenylphosphoryl azide (DPPA), to yield, after selective deprotection of the N-terminal amine function, the compound of formula (IV) : 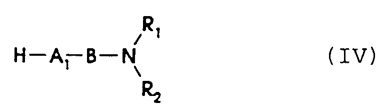 wherein A₁, B, R₁ and R₂ are as defined for formula (I),
which compound of formula (IV) is reacted with a compound of formula (V) : 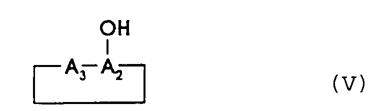 wherein A₂ and A₃ are as defined for formula (I), (the compound of formula (V) itself being obtained by conventional peptide coupling of protected optically pure amino acids A₃-OH and A₂-OH, cyclisation to form the corresponding diketopiperazine in basic medium, deprotection and purification), in the presence of a conventional coupling reagent for peptide synthesis described above, to yield a compound of formula (I/a) : 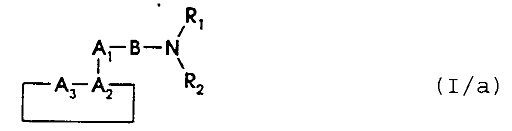 wherein A₁, A₂, A₃, B, R₁ and R₂ are as defined for formula (I),
it being possible for the radical A₁, when it represents a tryptophan residue, optionally to be protected by an appropriate reagent that contains the radical Q defined for formula (I),
which compound of formula (I/a), the tryptophan residue of which is optionally protected :
is purified by a conventional purification technique,
is converted, if desired, into its addition salts with a pharmaceutically acceptable acid or base,
which when they contain a psuedopeptide bond -CH₂-NH- or -CH₂-S- are prepared according to the process described above,
the introduction of the bond -CH₂-NH- being effected by preparing in solution the aldehyde P₁-NH-CHR-CHO (P₁ = a protecting group selected from Boc, Fmoc, Z) and condensing the aldehyde with the growing peptide chain either in solid phase or in solution,
the introduction of the bond -CH₂-S- being effected by condensing the thiol of formula P₁-NH-CHR-CH₂SH, prepared from the corresponding alcohol, with the C-terminal desamino-peptide bromide.

2. Process for the preparation of a compound according to claim 1 which is 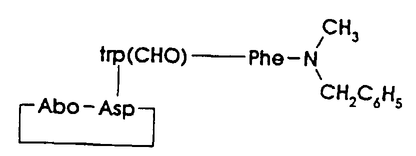 the enantiomers, diastereoisomers and epimers thereof.

3. Process for the preparation of a compound according to claim 1 which is 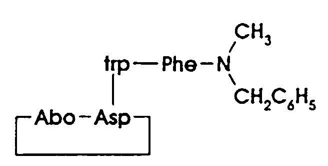 the enantiomers, diastereoisomers and epimers thereof.

4. Process for the preparation of a compound according to claim 1 which is 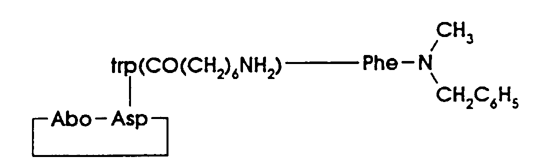 the enantiomers, diastereoisomers and epimers thereof and also the ammonium salts thereof.

5. Process for the preparation of a compound according to claim 1 which is 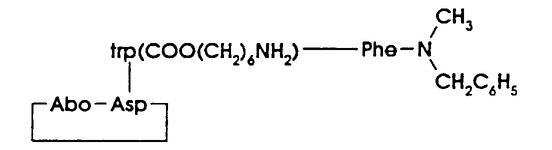 the enantiomers, diastereoisomers and epimers thereof and also the ammonium salts thereof.

6. Process for the preparation of pharmaceutical compositions comprising as active ingredient at least one compound according to any one of claims 1 to 5, alone or in combination with one or more pharmaceutically acceptable, inert, non-toxic excipients or carriers.

7. Process for the preparation of pharmaceutical compositions according to claim 6 comprising at least one active ingredient according to any one of claims 1 to 5 for use as antagonists of substance P in the treatment of pain, inflammation, gastrointestinal disorders, asthma, allergies, and disorders of the central nervous system.

## Patentansprüche (Patentansprüche für folgende(n) Vertragsstaat(en): AT, BE, CH, DE, DK, FR, GB, IT, LI, LU, NL, PT, SE)

1. Verbindungen der Formel (I): 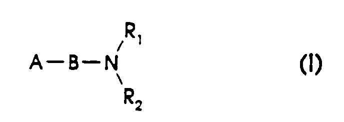 in der:
R₁ und R₂, die gleichartig oder verschieden sind, ein Wasserstoffatom, eine geradkettige oder verzweigte (C₁-C₆)-Alkylgruppe, eine (C₃-C₇)-Cycloalkylgruppe oder eine Benzylgruppe darstellen,
B einen Rest einer aromatischen Aminosäure ausgewählt aus Phenylalanin (Phe), Tyrosin (Tyr), (1,2,3,4)-Tetrahydroisochinolin-3-carbonyl (Tic), Tryptophan (Trp), Tryptophan geschützt durch eine Formylgruppe (Trp (CHO)) oder Pyridinylalanin (Pya) darstellt,
A einen Peptidrest der Formel: 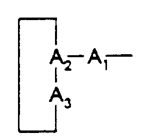 darstellt, in der:
A₁ eine Bindung, einen 2-Azabicyclo[2.2.2]octan-3-carbonylrest (Abo), Leu-cinrest (Leu), β-Naphthylalaninrest (Nal), Tryptophanrest (Trp) oder durch eine Gruppe Q geschützten Tryptophanrest (Trp(Q)) darstellt, worin
Q die Gruppe
-X-(CH₂)ₙ-R'
bedeutet, in der
X eine Bindung, -CO- oder -COO-,
n eine ganze Zahl mit einem Wert von 0 bis 10 und
R' ein Wasserstoffatom, eine geradkettige oder verzweigte (C₁-C₁₀)-Al-kylgruppe, eine Benzylgruppe, ene 9-Fluorenylmethylgruppe, -NH₂, -COOH oder -COOR" (worin R" für eine geradkettige oder verzweigte (C₁-C₆)-Alkylgruppe steht), bedeuten,
A₂ einen Asparaginsäurerest (Asp), und
A₃ einen (1,2,3,4)-Tetrahydroisochinolin-3-carbonylrest (Tic), einen 2-Azabicyclo[2.2.2]octan-3-carbonylrest (Abo) oder einen Methylphenylalaninrest (MePhe) bedeuten,
wobei es sich versteht, daß die Peptidbindung (-CO-NH-) zwischen A₁ und A₂ oder zwischen A₂ und B dann, wenn A₁ eine Bindung darstellt, durch eine Pseudopeptidbindung ausgewählt aus -CH₂-NH- oder -CH₂-S- ersetzt sein kann, deren Enantiomere, Diastereoisomere und Epimere sowie deren Additionssalze mit einer pharmazeutisch annehmbaren Säure oder Base,
wobei es sich versteht, daß jede Aminosäure der Peptidsequenz optisch rein ist und das α-Kohlenstoffatom einer jeden Aminosäure in der Konfiguration D oder L vorliegen kann.

2. Verbindung nach Anspruch 1, nämlich 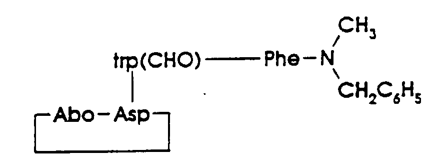 deren Enantiomere, Diastereoisomere und Epimere.

3. Verbindung nach Anspruch 1, nämlich 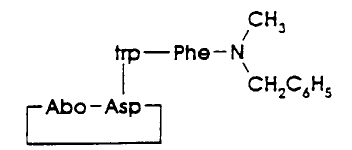 deren Enantiomere. Diastereoisomere und Epimere.

4. Verbindung nach Anspruch 1, nämlich 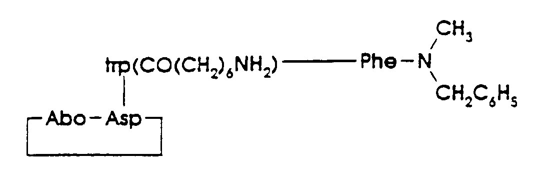 deren Enantiomere, Diastereoisomere und Epimere sowie deren Ammoniumsalze.

5. Verbindung nach Anspruch 1, nämlich 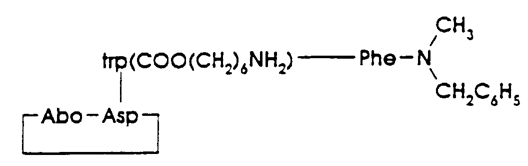 deren Enantiomere, Diastereoisomere und Epimere sowie deren Ammoniumsalze.

6. Verfahren zur Herstellung der Verbindungen der Formel (I) nach Anspruch 1, **dadurch gekennzeichnet**, daß man ein optisch reines Amid der Formel (II): 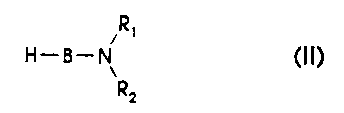 in der B, R₁ und R₂ die gleiche Bedeutung besitzen wie in der Formel (I), mit einer zweiten optisch reinen Aminosäure, deren N-terminale Aminogruppe geschützt ist, der Formel (III):
P₁-A₁-OH (III)
in der:
P₁ eine Schutzgruppe ausgewählt aus Benzyl-oxycarbonyl (Z), tert.-Butyloxycarbonyl (Boc) und 9-Fluorenyl-methylcarbonyl (Fmoc) darstellt und
A₁ die gleiche Bedeutung besitzt wie in der Formel (I),
in Gegenwart eines klassischen Kupplungsreagens der Peptidsynthese ausgewählt aus dem Paar Dicyclohexylcarbodiimid (DCC) - Hydroxybenzotriazol (HOBT), Benzotriazol-1-oxytris(dimethylamino)-phosphoniumhexafluorphosphat (BOP) und schließlich Diphenylphosphorylazid (DPPA) umsetzt, so daß man nach der selektiven Abspaltung der Schutzgruppe von der N-terminalen Aminogruppe die Verbindung der Formel (IV) erhält: 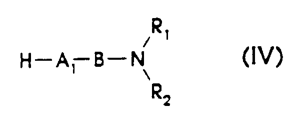 in der A₁, B, R₁ und R₂ die gleichen Bedeutungen besitzen wie in der Formel (I),
welche Verbindung der Formel (IV) man mit einer Verbindung der Formel (V): 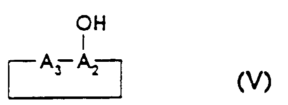 in der A₂ und A₃ die gleiche Bedeutung besitzen wie in der Formel (I), (welches Derivat der Formel (V) seinerseits durch klassische Peptidkupplung der geschützten, optisch reinen Aminosäuren A₃-OH und A₂-OH, Cyclisierung zu dem entsprechenden Diketopiperazin in basischem Medium. Abspaltung der Schutzgruppen und Reinigung erhalten worden ist), in Gegenwart eines klassischen Kupplungsreagens für die Peptidsynthese, wie es oben beschrieben worden ist, umsetzt, so daß man eine Verbindung der Formel (I/a) erhält: 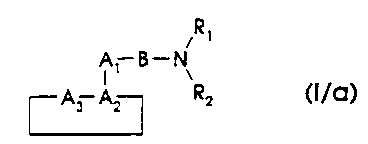 in der A₁, A₂, A₃, B, R₁ und R₂ die gleiche Bedeutung besitzen wie in der Formel (I),
wobei der Rest A₁, wenn er einen Tryptophanrest darstellt, gegebenenfalls mit einem geeigneten Reagens, das den Rest Q enthält, wie er für die Formel (I) definiert worden ist, geschützt werden kann.
welche Verbindung der Formel (I/a), deren Tryptophanrest gegebenenfalls geschützt ist, man
mit Hilfe einer klassischen Reinigungsmethode reinigt,
gegebenenfalls mit einer pharmazeutisch annehmbaren Säure oder Base in die Additionssalze umwandelt,
welche, wenn sie eine Pseudopeptidbindung -CH₂-NH- oder -CH₂-S- enthalten, gemäß dem oben beschriebenen Verfahren hergestellt werden,
wobei die Einführung der -CH₂-NH-Bindung dadurch erfolgt, daß man den Aldehyd P₁-NH-CHR-CHO (worin P₁ eine aus Boc, Fmoc und Z ausgewählte Schutzgruppe darstellt) in Lösung herstellt und entweder auf fester Phase oder in Lösung mit der wachsenden Peptidkette kondensiert und
die Einführung der -CH₂-S-Bindung dadurch erfolgt, daß man das Thiol der Formel P₁-NH-CHR-CH₂-SH, welches man ausgehend von dem entsprechenden Alkohol hergestellt hat, mit dem C-terminalen Bromid des Desaminopeptids kondensiert.

7. Pharmazeutische Zubereitungen enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien oder Bindemitteln.

8. Pharmazeutische Zubereitung nach Anspruch 7, enthaltend mindestens einen Wirkstoff nach einem der Ansprüche 1 bis 5 zur Verwendung als Antagonisten der Substanz P bei der Behandlung von Schmerzen, Entzündungen, Magen-Darm-Störungen, Asthma, Allergien und Erkrankungen des Zentralnervensystems.

## Patentansprüche (Patentansprüche für folgende(n) Vertragsstaat(en): GR, ES)

1. Verfahren zur Herstellung der Verbindungen der Formel (I): 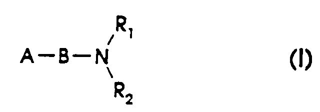 in der:
R₁ und R₂, die gleichartig oder verschieden sind, ein Wasserstoffatom, eine geradkettige oder verzweigte (C₁-C₆)-Alkylgruppe, eine (C₃-C₇)-Cycloalkylgruppe oder eine Benzylgruppe darstellen,
B einen Rest einer aromatischen Aminosäure ausgewählt aus Phenylalanin (Phe), Tyrosin (Tyr), (1,2,3,4)-Tetrahydroisochinolin-3-carbonyl (Tic), Tryptophan (Trp), Tryptophan geschützt durch eine Formylgruppe (Trp (CHO)) oder Pyridinylalanin (Pya) darstellt,
A einen Peptidrest der Formel: 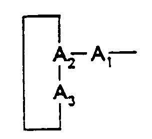 darstellt, in der:
A₁ eine Bindung, einen 2-Azabicyclo[2.2.2]octan-3-carbonylrest (Abo), Leucinrest (Leu), β-Naphthylalaninrest (Na1), Tryptophanrest (Trp) oder durch eine Gruppe Q geschützten Tryptophanrest (Trp(Q)) darstellt, worin
Q die Gruppe
-X-(CH₂)ₙ-R'
bedeutet, in der
X eine Bindung, -CO- oder -COO-,
n eine ganze Zahl mit einem Wert von 0 bis 10 und
R' ein Wasserstoffatom, eine geradkettige oder verzweigte (C₁-C₁₀)-Alkylgruppe, eine Benzylgruppe, ene 9-Fluorenylmethylgruppe, -NH₂, -COOH oder -COOR" (worin R" für eine geradkettige oder verzweigte (C₁-C₆)-Alkylgruppe steht), bedeuten,
A₂ einen Asparaginsäurerest (Asp), und
A₃ einen (1,2,3,4)-Tetrahydroisochinolin-3-carbonylrest (Tic), einen 2-Azabicyclo[2.2.2]octan-3-carbonylrest (Abo) oder einen Methylphenylalaninrest (MePhe) bedeuten,
wobei es sich versteht, daß die Peptidbindung (-CO-NH-) zwischen A₁ und A₂ oder zwischen A₂ und B dann, wenn A₁ eine Bindung darstellt, durch eine Pseudopeptidbindung ausgewählt aus -CH₂-NH- oder -CH₂-S- ersetzt sein kann. von deren Enantiomeren, Diastereoisomeren und Epimeren sowie von deren Additionssalzen mit einer pharmazeutisch annehmbaren Säure oder Base, wobei es sich versteht, daß jede Aminosäure der Peptidsequenz optisch rein ist und das α-Kohlenstoffatom jeder Aminosäure in der Konfiguration D oder L vorliegen kann, **dadurch gekennzeichnet**, daß man ein optisch reines Amid der Formel (II): 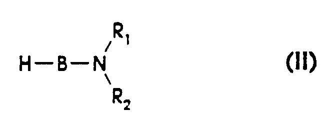 in der B, R₁ und R₂ die gleiche Bedeutung besitzen wie in der Formel (I), mit einer zweiten optisch reinen Aminosäure, deren N-terminale Aminogruppe geschützt ist, der Formel (III):
P₁-A₁-OH (III)
in der:
P₁ eine Schutzgruppe ausgewählt aus Benzyl-oxycarbonyl (Z), tert.-Butyloxycarbonyl (Boc) und 9-Fluorenyl-methylcarbonyl (Fmoc) darstellt und
A₁ die gleiche Bedeutung besitzt wie in der Formel (I),
in Gegenwart eines klassischen Kupplungsreagens der Peptidsynthese ausgewählt aus dem Paar Dicyclohexylcarbodiimid (DCC) - Hydroxybenzotriazol (HOBT), Benzotriazol-1-oxytris(dimethylamino)-phosphoniumhexafluorphosphat (BOP) und schließlich Diphenylphosphorylazid (DPPA) umsetzt,
so daß man nach der selektiven Abspaltung der Schutzgruppe von der N-terminalen Aminogruppe die Verbindung der Formel (IV) erhält: 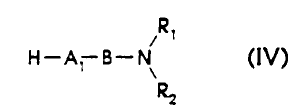 in der A₁, B, R₁ und R₂ die gleichen Bedeutungen besitzen wie in der Formel (I),
welche Verbindung der Formel (IV) man mit einer Verbindung der Formel (V): 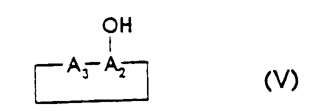 in der A₂ und A₃ die gleiche Bedeutung besitzen wie in der Formel (I), (welches Derivat der Formel (V) seinerseits durch klassische Peptidkupplung der geschützten, optisch reinen Aminosäuren A₃-OH und A₂-OH, Cyclisierung zu dem entsprechenden Diketopiperazin in basischem Medium, Abspaltung der Schutzgruppen und Reinigung erhalten worden ist), in Gegenwart eines klassischen Kupplungsreagens für die Peptidsynthese, wie es oben beschrieben worden ist, umsetzt, so daß man eine Verbindung der Formel (I/a) erhält: 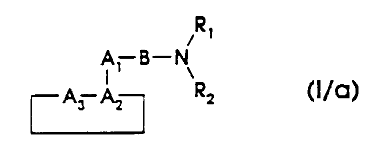 in der A₁, A₂, A₃, B, R₁ und R₂ die gleiche Bedeutung besitzen wie in der Formel (I),
wobei der Rest A₁, wenn er einen Tryptophanrest darstellt, gegebenenfalls mit einem geeigneten Reagens, das den Rest Q enthält, wie er für die Formel (I) definiert worden ist, geschützt werden kann,
welche Verbindung der Formel (I/a), deren Tryptophanrest gegebenenfalls geschützt ist, man
mit Hilfe einer klassischen Reinigungsmethode reinigt,
gegebenenfalls mit einer pharmazeutisch annehmbaren Säure oder Base in die Additionssalze umwandelt,
welche, wenn sie eine Pseudopeptidbindung-CH₂-NH- oder -CH₂-S- enthalten, gemäß dem oben beschriebenen Verfahren hergestellt werden.
wobei die Einführung der -CH₂-NH-Bindung dadurch erfolgt, daß man den Aldehyd P₁-NH-CHR-CHO (worin P₁ eine aus Boc, Fmoc und Z ausgewählte Schutzgruppe darstellt) in Lösung herstellt und entweder auf fester Phase oder in Lösung mit der wachsenden Peptidkette kondensiert und
die Einführung der -CH₂-S-Bindung dadurch erfolgt, daß man das Thiol der Formel P₁-NH-CHR-CH₂-SH, welches man ausgehend von dem entsprechenden Alkohol hergestellt hat, mit dem C-terminalen Bromid des Desaminopeptids kondensiert.

2. Verfahren zur Herstellung der Verbindung nach Anspruch 1, nämlich 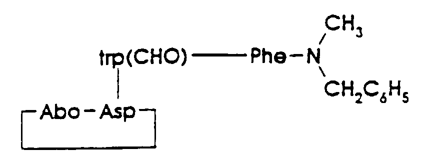 von deren Enantiomeren, Diastereoisomeren und Epimeren.

3. Verfahren zur Herstellung der Verbindung nach Anspruch 1, nämlich 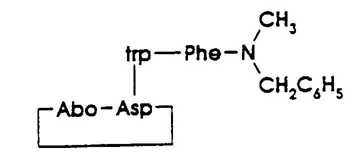 von deren Enantiomeren, Diastereoisomeren und Epimeren.

4. Verfahren zur Herstellung der Verbindung nach Anspruch 1, nämlich 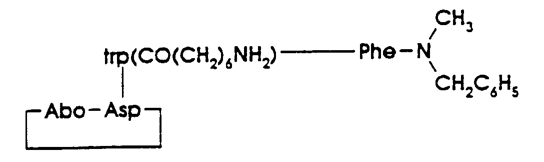 von deren Enantiomeren, Diastereoisomeren und Epimeren sowie von deren Ammoniumsalzen.

5. Verfahren zur Herstellung der Verbindung nach Anspruch 1, nämlich 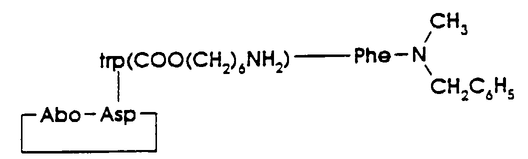 von deren Enantiomeren, Diastereoisomeren und Epimeren sowie von deren Ammoniumsalzen.

6. Verfahren zur Herstellung von pharmazeutischen Zubereitungen, die als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien oder Bindemitteln enthalten.

7. Verfahren zur Herstellung von pharmazeutischen Zubereitungen nach Anspruch 6, die mindestens einen Wirkstoff gemäß einem der Ansprüche 1 bis 5 enthalten, zur Verwendung als Antagonisten der Substanz P bei der Behandlung von Schmerzen, Entzündungen, Magen-Darm-Störungen, Asthma. Allergien und Erkrankungen des Zentralnervensystems.
